# EUROPEAN PATENT SPECIFICATION

(11) **EP 4 212 506 B1**
(45) Date of publication and mention of the grant of the patent: **07.10.2026**
(21) Application number: 21892255.7
(22) Date of filing: 05.11.2021
(51) Int. Cl.: C07C 51/377, C07C 51/43, C07C 59/08, B01J 27/18, C07C 57/04, B01J 35/40, B01J 35/55

(54) **A METHOD FOR PREPARING ACRYLIC ACID**
EIN VERFAHREN ZUR HERSTELLUNG VON ACRYLSÄURE
UN PROCÉDÉ DE PRÉPARATION D'ACIDE ACRYLIQUE

(30) Priority: 11.11.2020 KR 20200150320; 04.11.2021 KR 20210150793
(43) Date of publication of application: 19.07.2023
(73) Proprietor: LG Chem, Ltd., Seoul 07336 (KR)
(72) Inventor: CHOI, Inho, Daejeon 34122 (KR); JEONG, Hoiin, Daejeon 34122 (KR); IM, Yong O, Daejeon 34122 (KR); LEE, Jiyoung, Daejeon 34122 (KR); HONG, Daeho, Daejeon 34122 (KR)
(74) Representative: Goddar, Heinz J.
(86) International application number: PCT/KR2021/016059
(87) International publication number: WO 2022/103087

(56) References cited:
- WO-A1-2012/156921
- WO-A1-2012/156921
- JP-A- 2016 175 840
- US-A1- 2012 277 467
- US-A1- 2013 274 094
- US-A1- 2013 274 515
- US-A1- 2013 274 515

## Description

### [TECHNICAL FIELD]

The present disclosure relates to a method for preparing acrylic acid, and more particularly, to a method for preparing acrylic acid by dehydrating a lactic acid molecule.

### [BACKGROUND ART]

Acrylic acid is an organic compound having both a carboxylic acid and an unsaturated double bond in a molecule, is very simple in its structure and can be polymerized while being converted into various materials, and thus is used in various industrial fields.

Specifically, acrylic acid is used as polyacrylic acid, dots, adhesives, paints, etc. required for the production of a superabsorbent polymer, or can be used as a raw material for preparing other types of acrylate-based monomers, or may be used as a raw material for polymerizing with various other monomers such as acrylamide, acrylonitrile, styrene, and alpha olefin.

Such acrylic acid is generally prepared using propylene produced in the process of refining and separating a crude oil, such as naphtha cracking.

However, recently, as high attention is focused on crude oil depletion and environmental problems, interest in a method for preparing acrylic acid using an eco-friendly raw material is growing.

US 2013/274515 A1 discloses a method for preparing acrylic acid by dehydrating a lactic acid solution. The process comprises the steps of providing a lactic acid aqueous solution and mixing the lactic acid aqueous solution with an inert gas. This mixture is evaporated and dehydrated using a dehydration catalyst to prepare acrylic acid. After cooling, the acrylic acid is recovered. Temperatures in the evaporation step, the dehydration step and the acrylic acid recovery step may differ from each other.

### [DETAILED DESCRIPTION OF THE INVENTION]

### [Technical Problem]

It is one object of the present disclosure to provide a method capable of preparing acrylic acid from lactic acid with high conversion rate and yield.

### [Technical Solution]

Provided herein is a method for preparing acrylic acid, comprising the steps of: a first step of supplying an aqueous lactic acid solution to a reactor using a carrier gas; a second step of vaporizing the aqueous lactic acid solution; a third step of bringing the vaporized lactic acid molecule into contact with a dehydration catalyst; and a fourth step of obtaining acrylic acid, wherein the temperatures of the second to fourth steps are each independently adjusted, wherein
the first to fourth steps are performed using a reactor equipped with a single reaction tube and a heating unit;
the single reaction tube comprises a supply unit to which an aqueous lactic acid solution is supplied, a vaporization unit that vaporizes the aqueous lactic acid solution, a catalyst unit that brings the vaporized lactic acid molecule into contact with a dehydration catalyst; and a discharge unit that discharges acrylic acid;
the heating unit comprises a first heating unit that heats the vaporization unit in a shape wrapping the single reaction tube, a second heating unit that is discontinuous with the first heating unit and heats a boundary portion between the vaporization unit and the catalyst unit and a front end of the catalyst unit, and a third heating unit which is discontinuous with the second heating unit and heats the rear end of the catalyst unit, and
   the temperatures of the second to fourth steps are each independently adjusted by the heating unit, and
a set temperature of the second heating unit is higher than a set temperature of the third heating unit.

According to an embodiment of the present disclosure, the second step may be performed under a temperature condition of 200°C to 290°C, preferably under a temperature condition of 200°C or more, or about 230°C or more, or about 250°C or more and 290°C or less, or about 270°C or less, or about 260°C

Further, the second step may be performed in the presence of quartz.

According to another embodiment of the present disclosure, the third step may be performed under a temperature condition of more than 350°C and 400°C or less, preferably under a temperature condition of more 350°C, or about 355°C or more, or about 360°C or more, and 400°C or less, or about 390°C or less, or about 380°C or less.

Further, the dehydration catalyst may include at least one selected from the group consisting of a calcium phosphate-based catalyst, a sodium phosphate-based catalyst, and an aluminum phosphate-based catalyst.

According to another embodiment of the present disclosure, in the first step, the aqueous lactic acid solution may be supplied at a flow rate of 0.01 to 10 /hour, or at a flow rate of about 0.1 to about 5 /hour, or about 0.1 to about 1 /hour, based on a supply weight of lactic acid relative to a weight of the catalyst.

In this case, a concentration of the aqueous lactic acid solution in the first step may be 10 to 80 wt%.

According to the present disclosure, the first to fourth steps are performed using a reactor equipped with a single reaction tube and a heating unit; the single reaction tube comprises a supply unit to which an aqueous lactic acid solution is supplied, a vaporization unit that vaporizes the aqueous lactic acid solution, a catalyst unit that brings the vaporized lactic acid molecule into contact with a dehydration catalyst; and a discharge unit that discharges acrylic acid; the heating unit comprises a first heating unit that heats the vaporization unit in a shape wrapping the single reaction tube, a second heating unit that is discontinuous with the first heating unit and heats a boundary portion between the vaporization unit and the catalyst unit and a front end of the catalyst unit, and a third heating unit this is discontinuous with the second heating unit and heats the rear end of the catalyst unit, and the temperatures of the second to fourth steps are each independently adjusted by the heating unit.

In this case, the first heating unit may be heated so that the inside of the vaporization unit of the single reaction tube is maintained at a temperature condition of 200°C to 290°C. This is a temperature condition of the second step described above, and specifically may refer to a temperature condition of 200 °C to 290°C, preferably a temperature condition of 200°C or more, or about 230°C or more, or about 250°C or more and 290°C or less, or about 270°C or less, or about 260°C.

Further, the second heating unit and the third heating unit may be heated so that the inside of the catalyst unit of the single reaction tube is maintained at a temperature condition of more than 350°C and 400°C or less. This is a temperature condition of the third step described above, and specifically may refer to a temperature condition of about more than 350°C and 400°C or less, preferably a temperature condition of more than 350°C, or about 355°C or more, or about 360°C or more, and 400°C or less, or about 390°C or less, or about 380°C or less.

Specifically, a set temperature of the second heating unit may be 15°C to 30°C higher than a set temperature of the third heating unit

As used herein, the terms "a first," "a second," etc. are used herein to explain various constitutional elements, and these terms are used only to distinguish one constitutional element from another constitutional element.

The technical terms used herein is only to explain exemplary embodiments and is not intended to limit the scope of the present disclosure.

The singular forms "a," "an" and "the" are intended to include plural forms, unless the context clearly indicates otherwise.

It should be understood that the terms "comprise," "include", "have", etc. are used herein to specify the presence of stated features, integers, steps, components or combinations thereof, but do not preclude the presence or addition of one or more other features, integers, steps, components, or combinations thereof.

Also, as used herein, in case a layer or an element is mentioned to be formed "on" or "above" layers or elements, it means that the layer or element is directly formed on the layers or elements, or it means that other layers or elements may be additionally formed between the layers, on a subject, or on a substrate.

Although the present disclosure may have various forms and various modifications may be made thereto, specific examples will be exemplified and explained in detail below. However, it is not intended to limit the present disclosure to specific disclosure, and it should be understood that the present disclosure includes all the modifications, equivalents or replacements thereof without departing from the spirit and technical scope of the present disclosure.

Now, the present disclosure will be described in more detail.

According to one aspect of the present disclosure, there is provided a method for preparing acrylic acid, comprising the steps of: a first step of supplying an aqueous lactic acid solution to a reactor using a carrier gas; a second step of vaporizing the aqueous lactic acid solution; a third step of bringing the vaporized lactic acid molecule into contact with a dehydration catalyst; and a fourth step of obtaining acrylic acid, wherein the temperatures of the second to fourth steps are each independently adjusted, wherein
the first to fourth steps are performed using a reactor equipped with a single reaction tube and a heating unit;
the single reaction tube comprises a supply unit to which an aqueous lactic acid solution is supplied, a vaporization unit that vaporizes the aqueous lactic acid solution, a catalyst unit that brings the vaporized lactic acid molecule into contact with a dehydration catalyst; and a discharge unit that discharges acrylic acid;
the heating unit comprises a first heating unit that heats the vaporization unit in a shape wrapping the single reaction tube, a second heating unit that is discontinuous with the first heating unit and heats a boundary portion between the vaporization unit and the catalyst unit and a front end of the catalyst unit, and a third heating unit which is discontinuous with the second heating unit and heats the rear end of the catalyst unit, and
   the temperatures of the second to fourth steps are each independently adjusted by the heating unit, and
a set temperature of the second heating unit is higher than a set temperature of the third heating unit.

The present inventors have found that in a series of reactions to obtain acrylic acid by subjecting vaporized lactic acid molecules to a dehydration reaction in the presence of a catalyst, when the temperature of each step is independently adjusted by subdividing the vaporization step and the dehydration step, the efficiency of the reaction can be increased while reducing the generation of by-products, and the yield of acrylic acid and the conversion rate of lactic acid can be dramatically improved, thereby completing the present disclosure.

The dehydration reaction that proceeds for the vaporized lactic acid molecule in the presence of a catalyst can be represented by the following reaction mechanism.

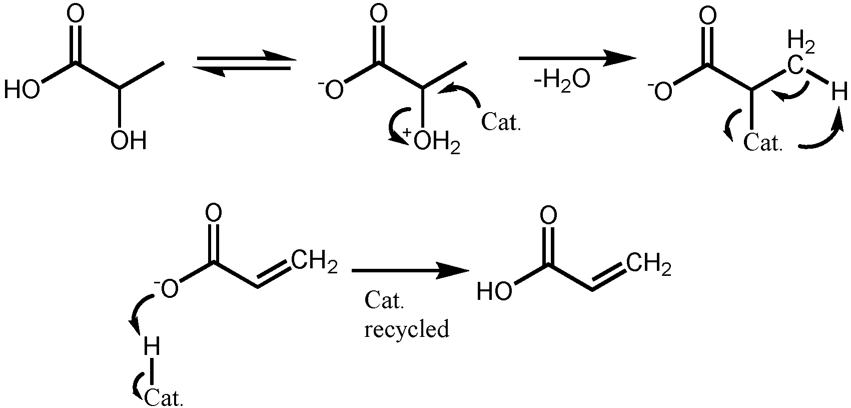

That is, the dehydration reaction of the lactic acid molecule can be explained as follows. A hydroxyl group linked to a carbonyl alpha position of the lactic acid molecule is released by a catalyst, hydrogen linked to a carbonyl beta position is also removed by a catalyst to form an acrylic acid anion, and then the hydrogen of the catalyst is linked to a carboxylate anion of acrylic acid to form acrylic acid.

It is known that such a reaction proceeds well for vaporized lactic acid molecules in the presence of a catalyst, but it competes with reactions that form by-products other than acrylic acid, such as i) an aldehyde formation reaction by decarboxylation or decarbonylation, ii) a propanoic acid formation reaction by reduction of acrylic acid, iii) a pentanedione formation reaction by condensation, and iv) an auto-esterification by dimerization, and the like, and therefore, the reaction conditions thereof need to be finely adjusted.

First, the reactants in the first step, i.e., lactic acid supplied as a feed, are in the form of an aqueous lactic acid solution, which may preferably be in a concentration range of about 10 to about 80 wt%.

When the concentration of lactic acid is too low, there is a problem that the efficiency of the vaporization reaction in the vaporization step and the efficiency of a subsequent lactic acid dehydration reaction become too low. When the concentration of lactic acid is too high, the content of oligomers such as dimers in the aqueous lactic acid solution increases and thus the efficiency of the lactic acid dehydration reaction of by-products may be lowered, which may cause a problem that the production of by-products is promoted.

And, in the first step, the aqueous lactic acid solution may be supplied at a flow rate of 0.01 to 10 /hour, or 0.1 to about 5 /hour, or about 0.1 to about 1 /hour, based on the supply weight of lactic acid relative to the weight of the catalyst.

When the supply amount of an aqueous lactic acid solution is too small, the residence time of lactic acid at high temperature increases, the rate of loss due to thermal decomposition increases. In addition, there may be a problem in that other side reactions increase. When the supply amount of an aqueous lactic acid solution is too large, lactic acid is not sufficiently vaporized by a heat source, and the temperature of the vaporization unit and the upper end of a catalyst layer is lowered, which may cause a problem that catalyst performance is deteriorated.

And, lactic acid supplied as a feed may be supplied by a carrier gas. As the carrier gas used at this time, an inert gas that does not affect the vaporization reaction or the dehydration reaction, such as nitrogen or a group 18 gas, can be used.

The flow rate of the carrier gas used for the reaction may be about 1 to about 1000 times, or about 10 to about 500 times, or about 20 to about 300 times the supply amount of the aqueous lactic acid solution.

According to an embodiment of the present disclosure, the second step, that is, the vaporization reaction of lactic acid molecules, is performed under a temperature condition of 200°C to 290°C, preferably under a temperature condition of 200°C or more, or about 230°C or more, or about 250°C or more and 290°C or less, or about 270°C or less, or about 260°C.

When the temperature of the vaporization reaction is too low, the efficiency of the vaporization reaction is lowered, which may lead to a problem that the efficiency of a subsequent dehydration reaction is also lowered, and when the temperature of the vaporization reaction is too high, decarboxylation or decarbonylation proceeds more remarkably in the vaporized lactic acid molecule, which may cause a problem that production of aldehyde is promoted.

Further, the second step may be performed in the presence of quartz. Specifically, the quartz may be in the form of quartz wool with a large surface area, or quartz particle.

That is, the lactic acid molecule supplied to the supply unit may be adsorbed onto the surface of the quartz wool or the like in the vaporization unit inside the reactor according to the flow of the carrier gas, and in this state, it may receive supply of heat from the quartz wool or the like to be vaporized.

And, as described above, the reactor used for this reaction may be provided with a single reaction tube and a heating unit, and the heating unit may be a shape wrapping the single reaction tube. In this case, the first heating unit for heating the vaporization unit may be heated so that the inside of the vaporization part of the single reaction tube is maintained at a temperature condition of 200 °C to 290°C This is the temperature condition of the second step described above, and specifically may refer to a temperature condition of 200°C to 290°C, preferably 200°C or more, or about 230°C or more, or about 250°C or more and 290°C or less, or about 270°C or less, or about 260°C.

However, throughout the specification, a temperature as a reaction condition of each reaction and a set temperature of the first to third heating units positioned at each site of the reactor may be different from each other. Specifically, the set temperature of the first to third heating units may be preferably set higher than the temperature of the respective corresponding reaction conditions. This may be because external gas and reactants are continuously supplied into a reactor according to the flow of the carrier gas, particularly, the temperature of the reactants supplied to the supply unit is commonly lower than the temperature of the vaporization unit, and in the vaporization unit, the temperature is continuously lowered due to the vaporization of lactic acid and water. Moreover, the temperature of the vaporization unit where the vaporization reaction proceeds is lower than the temperature of the catalyst unit where the dehydration reaction proceeds.

From this viewpoint, the first heating unit may be preferably set to a target temperature of the vaporization unit, that is, 15 to 30°C higher than the preferred temperature of the above-mentioned vaporization reaction.

Then, the gaseous reaction product containing the vaporized lactic acid single molecule continues to move to a catalyst unit where the catalyst exists according to the flow of the carrier gas, and can be charged into a dehydration reaction, that is, the third step.

According to another embodiment of the present disclosure, the third step is performed under a temperature condition of more than 350°C and 400°C or less, preferably under a temperature condition of more than 350°C, or about 355°C or more, or about 360 °C or more, and 400°C or less, or about 390°C or less, or about 380 °C or less.

When the temperature of the third step is too low, there may be a problem that the lactic acid conversion rate and the acrylic acid yield are greatly reduced, and when the temperature in step 3 is too high, i) an aldehyde formation reaction by decarboxylation or decarbonylation reaction, ii) a propanoic acid formation reaction by reduction of acrylic acid, iii) a pentanedione formation reaction by condensation, and the like are more promoted, which may cause a problem that by-products are increased.

And, as described above, the temperature of the catalyst unit as the dehydration reaction condition and the set temperature of the second and third heating units positioned at each site of the reactor may be different from each other.

Specifically, the second heating unit may be in the shape wrapping, in the interior of the reactor, i) a boundary portion between the vaporization unit and the catalyst unit and ii) a portion corresponding thereto in a single reaction tube for heating the front end of the catalyst unit. The third heating unit may be in the shape wrapping iii) a portion corresponding thereto in a single reaction tube for heating the rear end of the catalyst unit.

In addition, the second heating unit and the third heating unit may be heated so that the inside of the catalyst unit of the single reaction tube is maintained at a temperature of more than 350°C and 400°C or less. This is the temperature condition of the third step described above, and may refer to a temperature condition of more than 350°C and 400°C or less, preferably a temperature condition of more than 350°C, or about 355°C or more, or about 360°C or more, and 400°C or less, or about 390°C or less, or about 380°C or less.

In this case, the set temperature of the second heating unit is higher than the set temperature of the third heating unit, specifically, it may be preferable that the set temperature of the second heating unit is 15°C to 30°C higher than the set temperature of the third heating unit.

For example, it may be preferable that the second and third heating units are set higher than the target temperature of the catalyst unit, that is, 15°C to 30°C higher than the above-mentioned preferable temperature for the dehydration reaction. In particular, the second heating unit may be set to a higher temperature than the third heating unit, and for example, it may be set to 15°C to 30°C higher temperature.

As described above, due to the type of reaction in which external gas and reactants are continuously supplied into the reactor according to the flow of the carrier gas, the temperature at the front end of the vaporization unit and the catalyst unit is inevitably lowered continuously. This is because the temperature of the vaporization unit and the front end of the catalyst unit may be lowered, thereby greatly reducing the overall reaction efficiency.

In the conventional case, in order to prevent these problems, a method such as setting the overall temperature of the reactor higher or preheating the reactor before the reaction proceeds to reach a target temperature, and then charging to the reaction was used. However, in such a case, energy is wasted unnecessarily, and the efficiency of the reaction may be lowered due to the side reaction described above under high temperature conditions, whereas it is still difficult to solve the problem that the temperature at the front end of the catalyst unit is lowered.

In the case of the present disclosure, the temperature of the vaporization unit, the boundary portion between the vaporization unit and the catalyst unit, the front end of the catalyst unit, and the rear end of the catalyst unit are set differently according to the needs of each reaction. In particular, a separate heating unit, that is, a second heating unit, capable of supplying heat is positioned at the boundary portion between the vaporization unit and the catalyst unit, where the temperature condition changes rapidly, whereby the energy efficiency can be further increased and also the reaction efficiency can be improved.

In addition, the dehydration catalyst may include at least one selected from the group consisting of a calcium phosphate-based catalyst, a sodium phosphate-based catalyst, and an aluminum phosphate-based catalyst, and other reaction conditions can be used without any particular limitation as long as they are those generally used in the art to which the present disclosure pertains, and are not contrary to the content defined herein.

More specifically, the dehydration catalyst may include CaSO4/Na2SO4; Na4P2O7/CaSO4; Na4P2O7/Ca3(PO4)2; NaH2PO4-NaHCO3/SiO2; AlPO4-NH3; Ca3(PO4)2/CaSO4; Ca2P2O7; Ca5(PO4)3(OH) and the like.

### [Advantageous Effects]

According to the preparation method of the present disclosure, acrylic acid can be prepared from lactic acid with a high conversion rate and yield, and also energy consumption can be further reduced as compared with a conventional method.

### [DETAILED DESCRIPTION OF THE EMBODIMENTS]

Hereinafter, the action and effect of the invention will be described in more detail with reference to specific examples of the invention. However, these examples are presented for illustrative purposes only and the scope of the invention is not limited thereby in any way.

### <Example>

A reaction cylinder made of quartz with an inner diameter of 2,22 cm (7/8 inches) and a length of 860 mm was prepared as a single reaction tube.

A non-reactive glass tube and quartz wool were added so that quartz sand is not poured down in a region of about 150 mm to about 300 mm from the upper end of the single reaction tube, and quartz sand was filled therein to form a vaporization unit.

As the catalyst, a catalyst obtained by molding a calcium phosphate catalyst into a cylindrical pellet having a diameter of about 3 mm and a length of about 3 mm was used.

Then, a non-reactive glass tube and quartz wool were placed in a region of a length of about 300 mm from the lower end of the vaporized portion so that the catalyst was not poured down, and about 50 g of the catalyst was filled therein to form a catalyst unit.

A first heating unit having a length of about 200 mm was provided in a shape surrounding the entire region corresponding to the vaporization unit of the single reaction tube, from a position of about 100 mm downward from the top of the single reaction tube.

Alternatively, a second heating unit having a length of about 100 mm was provided in a shape surrounding a boundary portion between the vaporization unit and the catalyst unit of a single reaction tube, and a region corresponding to the front end of the catalyst unit, from the rear end of the first heating unit.

Alternatively, a third heating unit having a length of about 200 mm was provided in a shape surrounding a region corresponding to the rear end of the catalyst unit of the single reaction tube from the rear end of the second heating unit.

Alternatively, a fourth heating unit having a length of about 200 mm was provided in a shape surrounding a region corresponding to a discharge unit of the single reaction tube from the rear end of the second heating unit.

A thermocouple was provided at the front and rear ends of the catalyst unit of the single reaction tube so as to measure the internal temperature.

Nitrogen was used as a carrier gas, the flow rate was about 80 ml/min, and an aqueous lactic acid solution having a concentration of about 40 wt% was set at a flow rate of about 0.4 ml/min, and supplied to the reactor. The density of the supplied aqueous lactic acid solution was about 1.08 g/ml, the supply rate of the aqueous lactic acid solution was about 10.37 g/hour based on the amount of lactic acid, and it was calculated to be about 0.21 /hour for the reference weight (1 g) of the supplied catalyst (50 g).

The product sample obtained from the discharge unit was collected, cooled to about 4°C in a condenser, and collected in the liquid phase, and the amount of acrylic acid obtained was confirmed by HPLC.

During the reaction, the set temperatures of the first to fourth heating units were made respectively different from each other, and the temperatures of the front and rear ends of the catalyst unit were measured while performing the vaporization reaction and the dehydration reaction.

The measurement results are summarized in Table below.

**[Table 1]**

| | Set temperature for each region (1st, 2nd, 3rd, 4th heating unit) | Temperature of the front and rear ends of catalyst unit | Lactic acid conversion rate (%) | Acrylic acid yield (%) | Acetaldehyde yield (%) |
|---|---|---|---|---|---|
| Reference Example 1 | 250, 320, 350, 350 | 298, 349 | 47.7 | 14.2 | 15.3 |
| Reference Example 2 | 250, 330, 350, 350 | 317, 361 | 58.7 | 20.3 | 16.9 |
| Reference Example 3 | 250, 340, 350, 350 | 335, 364 | 76.9 | 22.3 | 18.1 |
| Comparative Example 1 | 300, 340, 350, 350 | 367, 370 | 97.0 | 29.1 | 30.8 |
| Comparative Example 2 | 350, 330, 350, 350 | 375, 370 | 100 | 25.7 | 40.0 |
| Example 1 | 250, 370, 350, 350 | 366, 370 | 92.6 | 40.1 | 19.2 |
| Example 2 | 250, 375, 350, 350 | 370, 370 | 94.0 | 48.1 | 20.9 |

Referring to Table 1, it can be clearly confirmed that the method for preparing acrylic acid according to an embodiment of the present disclosure can prepare acrylic acid from lactic acid with a high conversion rate and a high yield.

Further, it can be confirmed that when the temperature of the first heating unit (lactic acid vaporization, second stage) increases to 300°C or more as in Comparative Examples 1 and 2, the rate at which acetaldehyde is formed is higher than in Examples 1 and 2 even though the temperature of the front end of the catalyst unit was set similarly to Examples 1 and 2. This seems to be due to the fact that the temperature of lactic acid vaporization is too high, so that the decarboxylation reaction or decarbonylation reaction is more predominant than the dehydration reaction.

Comparing the results according to Examples and Comparative Examples, it can be confirmed that when the temperature of each step of the lactic acid vaporization reaction, such as a step of supplying an aqueous lactic acid solution to a reactor using a carrier gas, a step of vaporizing the aqueous lactic acid solution, a step of bring the vaporized lactic acid molecule into contact with a dehydration catalyst, and a step of obtaining acrylic acid, and the like as in an embodiment of the present disclosure is adjusted independently, side reactions such as acetaldehyde generation can be effectively suppressed while maximizing lactic acid conversion rate and acrylic acid yield.

## Claims

1. A method for preparing acrylic acid, comprising the steps of:
a first step of supplying an aqueous lactic acid solution to a reactor using a carrier gas;
a second step of vaporizing the aqueous lactic acid solution;
a third step of bringing the vaporized lactic acid molecule into contact with a dehydration catalyst; and
a fourth step of obtaining acrylic acid,
wherein temperatures of the second to fourth steps are each independently adjusted,
wherein
the first to fourth steps are performed using a reactor equipped with a single reaction tube and a heating unit;
the single reaction tube comprises a supply unit to which an aqueous lactic acid solution is supplied, a vaporization unit that vaporizes the aqueous lactic acid solution, a catalyst unit that brings the vaporized lactic acid molecule into contact with a dehydration catalyst; and a discharge unit that discharges acrylic acid;
the heating unit comprises a first heating unit that heats the vaporization unit in a shape wrapping the single reaction tube, a second heating unit that is discontinuous with the first heating unit and heats a boundary portion between the vaporization unit and the catalyst unit and a front end of the catalyst unit, and a third heating unit which is discontinuous with the second heating unit and heats the rear end of the catalyst unit, and
the temperatures of the second to fourth steps are each independently adjusted by the heating unit, and
a set temperature of the second heating unit is higher than a set temperature of the third heating unit.

2. The method for preparing acrylic acid according to claim 1, wherein:
the second step is performed under a temperature condition of 200°C to 290°C.

3. The method for preparing acrylic acid according to claim 1, wherein:
the second step is performed in the presence of quartz.

4. The method for preparing acrylic acid according to claim 1, wherein:
the third step is performed under a temperature condition of more than 350°C and 400°C or less.

5. The method for preparing acrylic acid according to claim 1, wherein:
the dehydration catalyst comprises at least one selected from the group consisting of a calcium phosphate-based catalyst, a sodium phosphate-based catalyst, and an aluminum phosphate-based catalyst.

6. The method for preparing acrylic acid according to claim 1, wherein:
in the first step, the aqueous lactic acid solution is supplied at a flow rate of 0.01 to 10 /hour, based on a supply weight of lactic acid relative to a weight of the catalyst.

7. The method for preparing acrylic acid according to claim 1, wherein:
a concentration of the aqueous lactic acid solution in the first step is 10 to 80 wt%.

8. The method for preparing acrylic acid according to claim 1, wherein:
the first heating unit is heated so that the inside of the vaporization unit of the single reaction tube is maintained at a temperature condition of 200°C to 290°C.

9. The method for preparing acrylic acid according to claim 1, wherein:
the second heating unit and the third heating unit are heated so that the inside of the catalyst unit of the single reaction tube is maintained at a temperature condition of more than 350°C and 400°C or less.

10. The method for preparing acrylic acid according to claim 1, wherein:
a set temperature of the second heating unit is 15°C to 30°C higher than a set temperature of the third heating unit.

## Patentansprüche

1. Verfahren zur Herstellung von Acrylsäure, umfassend die Schritte:
einen ersten Schritt des Zuführens einer wässrigen Milchsäurelösung zu einem Reaktor unter Verwendung eines Trägergases;
einen zweiten Schritt des Verdampfens der wässrigen Milchsäurelösung;
einen dritten Schritt des Inkontaktbringens des verdampften Milchsäuremoleküls mit einem Dehydratisierungskatalysator; und
einen vierten Schritt des Erhaltens von Acrylsäure,
wobei die Temperaturen des zweiten bis vierten Schritts jeweils unabhängig eingestellt werden,
wobei
der erste bis vierte Schritt unter Verwendung eines Reaktors durchgeführt werden, der mit einem einzelnen Reaktionsrohr und einer Heizeinheit ausgestattet ist;
das einzelne Reaktionsrohr eine Zuführeinheit, der eine wässrige Milchsäurelösung zugeführt wird, eine Verdampfungseinheit, die die wässrige Milchsäurelösung verdampft, eine Katalysatoreinheit, die das verdampfte Milchsäuremolekül mit einem Dehydratisierungskatalysator in Kontakt bringt; und eine Abführeinheit, die Acrylsäure abführt, umfasst;
die Heizeinheit eine erste Heizeinheit, die die Verdampfungseinheit in einer Form erwärmt, die das einzelne Reaktionsrohr umgibt, eine zweite Heizeinheit, die mit der ersten Heizeinheit diskontinuierlich ist und einen Grenzabschnitt zwischen der Verdampfungseinheit und der Katalysatoreinheit und ein vorderes Ende der Katalysatoreinheit erwärmt, und eine dritte Heizeinheit, die mit der zweiten Heizeinheit diskontinuierlich ist und das hintere Ende der Katalysatoreinheit erwärmt, umfasst, und
die Temperaturen des zweiten bis vierten Schritts jeweils unabhängig durch die Heizeinheit eingestellt werden, und
eine Solltemperatur der zweiten Heizeinheit höher als eine Solltemperatur der dritten Heizeinheit ist.

2. Verfahren zur Herstellung von Acrylsäure nach Anspruch 1, wobei:
der zweite Schritt unter einer Temperaturbedingung von 200 °C bis 290 °C durchgeführt wird.

3. Verfahren zur Herstellung von Acrylsäure nach Anspruch 1, wobei:
der zweite Schritt in Gegenwart von Quarz durchgeführt wird.

4. Verfahren zur Herstellung von Acrylsäure nach Anspruch 1, wobei:
der dritte Schritt unter einer Temperaturbedingung von mehr als 350 °C und 400 °C oder weniger durchgeführt wird.

5. Verfahren zur Herstellung von Acrylsäure nach Anspruch 1, wobei:
der Dehydratisierungskatalysator mindestens einen, ausgewählt aus der Gruppe, bestehend aus einem Katalysator auf Calciumphosphatbasis, einem Katalysator auf Natriumphosphatbasis und einem Katalysator auf Aluminiumphosphatbasis, umfasst.

6. Verfahren zur Herstellung von Acrylsäure nach Anspruch 1, wobei:
in dem ersten Schritt die wässrige Milchsäurelösung mit einer Fließgeschwindigkeit von 0,01 bis 10 /Stunde, bezogen auf ein Zuführgewicht von Milchsäure relativ zu einem Gewicht des Katalysators, zugeführt wird.

7. Verfahren zur Herstellung von Acrylsäure nach Anspruch 1, wobei:
eine Konzentration der wässrigen Milchsäurelösung in dem ersten Schritt 10 bis 80 Gew.-% beträgt.

8. Verfahren zur Herstellung von Acrylsäure nach Anspruch 1, wobei:
die erste Heizeinheit so erwärmt wird, dass das Innere der Verdampfungseinheit des einzelnen Reaktionsrohrs bei einer Temperaturbedingung von 200 °C bis 290 °C gehalten wird.

9. Verfahren zur Herstellung von Acrylsäure nach Anspruch 1, wobei:
die zweite Heizeinheit und die dritte Heizeinheit so erwärmt werden, dass das Innere der Katalysatoreinheit des einzelnen Reaktionsrohrs bei einer Temperaturbedingung von mehr als 350 °C und 400 °C oder weniger gehalten wird.

10. Verfahren zur Herstellung von Acrylsäure nach Anspruch 1, wobei:
eine Solltemperatur der zweiten Heizeinheit 15 °C bis 30 °C höher als eine Solltemperatur der dritten Heizeinheit ist.

## Revendications

1. Procédé de préparation d'acide acrylique, comprenant les étapes consistant en :
une première étape de fourniture d'une solution aqueuse d'acide lactique à un réacteur en utilisant un gaz vecteur ;
une deuxième étape de vaporisation de la solution aqueuse d'acide lactique ;
une troisième étape de mise en contact de la molécule d'acide lactique vaporisé avec un catalyseur de déshydratation ; et
une quatrième étape d'obtention d'acide acrylique,
dans lequel les températures de la deuxième à la quatrième étape sont chacune ajustées indépendamment,
dans lequel
la première à la quatrième étape sont effectuées en utilisant un réacteur équipé d'un tube de réaction unique et d'une unité de chauffage ;
le tube de réaction unique comprend une unité de fourniture à laquelle une solution aqueuse d'acide lactique est fournie, une unité de vaporisation qui vaporise la solution aqueuse d'acide lactique, une unité catalyseur qui met en contact la molécule d'acide lactique vaporisé avec un catalyseur de déshydratation ; et une unité de décharge qui décharge l'acide acrylique ;
l'unité de chauffage comprend une première unité de chauffage qui chauffe l'unité de vaporisation en une forme enveloppant le tube de réaction unique, une deuxième unité de chauffage qui est discontinue avec la première unité de chauffage et chauffe une partie de jonction entre l'unité de vaporisation et l'unité catalyseur et une extrémité avant de l'unité catalyseur, et une troisième unité de chauffage qui est discontinue avec la deuxième unité de chauffage et chauffe l'extrémité arrière de l'unité catalyseur, et
les températures de la deuxième à la quatrième étape sont chacune ajustées indépendamment par l'unité de chauffage, et
une température de consigne de la deuxième unité de chauffage est supérieure à une température de consigne de la troisième unité de chauffage.

2. Procédé de préparation d'acide acrylique selon la revendication 1, dans lequel :
la deuxième étape est effectuée à une condition de température de 200 °C à 290 °C.

3. Procédé de préparation d'acide acrylique selon la revendication 1, dans lequel :
la deuxième étape est effectuée en présence de quartz.

4. Procédé de préparation d'acide acrylique selon la revendication 1, dans lequel :
la troisième étape est effectuée à une condition de température supérieure à 350 °C et 400 °C ou moins.

5. Procédé de préparation d'acide acrylique selon la revendication 1, dans lequel :
le catalyseur de déshydratation comprend au moins l'un sélectionné parmi le groupe composé d'un catalyseur à base de phosphate de calcium, d'un catalyseur à base de phosphate de sodium et d'un catalyseur à base de phosphate d'aluminium.

6. Procédé de préparation d'acide acrylique selon la revendication 1, dans lequel :
à la première étape, la solution aqueuse d'acide lactique est fournie à un débit de 0,01 à 10/heure, sur une base du poids de fourniture d'acide lactique par rapport au poids du catalyseur.

7. Procédé de préparation d'acide acrylique selon la revendication 1, dans lequel :
une concentration de la solution aqueuse d'acide lactique à la première étape est de 10 à 80 % en poids.

8. Procédé de préparation d'acide acrylique selon la revendication 1, dans lequel :
la première unité de chauffage est chauffée de sorte que l'intérieur de l'unité de vaporisation du tube de réaction unique est maintenu à une condition de température de 200 °C à 290 °C.

9. Procédé de préparation d'acide acrylique selon la revendication 1, dans lequel :
la deuxième unité de chauffage et la troisième unité de chauffage sont chauffées de sorte que l'intérieur de l'unité catalyseur du tube de réaction unique est maintenu à une condition de température de plus de 350 °C et 400 °C ou moins.

10. Procédé de préparation d'acide acrylique selon la revendication 1, dans lequel :
une température de consigne de la deuxième unité de chauffage est de 15 °C à 30 °C plus élevée qu'une température de consigne de la troisième unité de chauffage.
